# EUROPEAN PATENT SPECIFICATION

(11) **EP 4 003 034 B1**
(45) Date of publication and mention of the grant of the patent: **05.11.2025**
(21) Application number: 20743615.5
(22) Date of filing: 14.07.2020
(51) Int. Cl.: A23C 9/12, A23C 21/02, A23L 33/21, C12P 19/14, A23L 2/60, C12P 19/04

(54) **FOOD INGREDIENT AND METHOD FOR PRODUCING THE SAME**
LEBENSMITTELZUTAT UND VERFAHREN ZUR HERSTELLUNG DAVON
INGRÉDIENT ALIMENTAIRE ET SON PROCÉDÉ DE PRODUCTION

(30) Priority: 26.07.2019 SG 10201906923U
(43) Date of publication of application: 01.06.2022
(60) Divisional application: 25204830.1
(73) Proprietor: Société des Produits Nestlé S.A., 1800 Vevey (CH)
(72) Inventor: LIANG, Youyun, SINGAPORE 618802 (SG); HOU, Yangfeng, Xiamen, Fujian 361111 (CN)
(74) Representative: Elleby, Gudrun
(86) International application number: PCT/EP2020/069884
(87) International publication number: WO 2021/018569

(56) References cited:
- WO-A1-2019/002304
- DUARTE P.M. TORRES ET AL: "Galacto-Oligosaccharides: Production, Properties, Applications, and Significance as Prebiotics", COMPREHENSIVE REVIEWS IN FOOD SCIENCE AND FOOD SAFETY, vol. 9, no. 5, 26 September 2010 (2010-09-26), pages 438 - 454, XP055099117, ISSN: 1541-4337, DOI: 10.1111/j.1541-4337.2010.00119.x
- ANONYMOUS: "Vivinal GOS Powder WPC - Product data sheet", 1 April 2019 (2019-04-01), pages 1 - 2, XP055722180, Retrieved from the Internet <URL:https://www.frieslandcampinaingredients.com/app/uploads/2019/04/PDS_ELN_Vivinal -GOS-Powder-WPC.pdf> [retrieved on 20200812]

## Description

### Field of the invention

The present invention relates to a food ingredient comprising galactooligosaccharides and a way of producing it.

### Background

Many food products traditionally comprise high amounts of sugar, which is important to achieve the sweet taste and texture the consumers expect of the product. For health reasons there is a desire to reduce the amount of sugar in many food products and to replace it with healthier ingredients like e.g. fibers, but to ensure the consumer liking of the product, it is desired to achieve this without sacrificing taste and texture. One solution is to use artificial sweeteners that provide sweetness without the energy contribution of sugar, but these do not contribute to the texture like sugar and furthermore, many consumers want to avoid artificial ingredients such as sweeteners. Food products such as e.g. cocoa and/or malt beverages traditionally comprise milk, which includes lactose, and sucrose to achieve the desired taste and texture. For the reasons given above, there is a desire to reduce the amount of sugar, preferably replacing it with healthier ingredients, keeping the taste and texture of the original product.

It is known that lactose can be polymerized by beta-galactosidase to produce galactooligosaccharide (GOS). GOS acts as prebiotics and is used as an ingredient primarily in infant formulae and baby food. The yield of GOS from polymerization of lactose is normally below 50%, often in the range of 10-30%.

WO2019/002304A1 relates to a hypoallergenic galacto-oligosaccharide nutritional ingredient, the method for producing it and its use in food and drink items.

### Summary of the invention

The inventors have found that a food ingredient with a specific combination of milk protein, sugars and GOS can be used in food products, such as cocoa and/or malt beverages, to replace skim milk powder or other milk ingredients as well as part of the sucrose normally added, to achieve similar taste and texture with reduced total sugar content and at the same time contribute prebiotic GOS to the product. Accordingly, the invention relates to a food ingredient comprising 20-40 % by dry weight of milk protein; and 35-55% by dry weight of a mixture of lactose, glucose, galactose and galactooligosaccharide, said mixture containing: 1-14% by weight of lactose, 15-29% by weight of glucose, 1-8% by weight of galactose and 70-90% by weight of galactooligosaccharide.

In a second aspect, the invention relates to a method for producing the food ingredient according to the invention, the method comprising:
a) providing an aqueous composition comprising 30-80% by weight of non-fat milk solids including lactose,
b) adding a beta-galactosidase to the composition, wherein the beta-galactosidase is one or more enzymes with enzymatic activity of enzyme class EC 3.2.1.23, which catalyses the hydrolysis of terminal non-reducing beta-D-galactose residues in beta-D-galactosides, as well as transgalactosylation by transferring a galactose moiety of a beta-D-galactoside to another sugar molecule,
c) allowing the beta-galactosidase to react for 10-240 minutes at 50-65°C; and
d) heat treating the composition after step c) at a temperature of 70-150°C for 10-150 seconds.

### Detailed description of the invention

### Definitions

By beta-galactosidase is understood one or more enzymes with enzymatic activity of enzyme class EC 3.2.1.23, also called beta-D-galactoside galactohydrolase, exo-(1->4)-beta-D-galactanase or lactase, which catalyses the hydrolysis of terminal non-reducing beta-D-galactose residues in beta-D-galactosides, as well as transgalactosylation by transferring a galactose moiety of a beta-D-galactoside to another sugar molecule.

EC (Enzyme Committee) numbers refer to the definition of enzymatic activity and nomenclature given by the Nomenclature Committee of the International Union of Biochemistry and Molecular Biology as in force on 3rd July 2019. Galactooligosaccharides (GOS) are oligosaccharides composed of different galactosyl residues (usually from 2 to 9 units) and a terminal glucose linked by β-glycosidic bonds, such as β-(1-2), β-(1-3), β-(1-4), and β-(1-6). GOS are normally produced through the enzymatic conversion of lactose.

### Food ingredient

In one aspect, the present invention relates to a food ingredient comprising 20-40% by dry weight of milk protein, as defined in the appended claims. By milk protein is meant any protein or combination of proteins derived from milk. The milk proteins may have been derived from milk in any suitable way. In a preferred embodiment, the food ingredient comprises 25-40%, more preferably 30-40% by dry weight of milk protein.

The food ingredient further comprises 35-55% by dry weight of a mixture of Lactose, Glucose, Galactose and GOS, said mixture containing: 1-14% by weight of lactose, 15-29% by weight of glucose, 1-8% by weight of galactose and 70-90% by weight of GOS. In a preferred embodiment, the food ingredient comprises 45-55% by dry weight of said mixture of Lactose, Glucose, Galactose and GOS; in a more preferred embodiment, the food ingredient comprises 50-55% by dry weight of said mixture of Lactose, Glucose, Galactose and GOS.

The food ingredient may further comprise 0.5-30% by dry weight of milk fat and/or minerals derived from milk, preferably 2-20%, more preferably 6-10% by dry weight of milk fat and/or minerals derived from milk. By milk fat is meant fat derived from milk in any suitable way. By milk derived minerals are meant minerals derived from milk in any suitable way, e.g. calcium, sodium, potassium, phosphorus and/or magnesium salts. Milk fat and/or milk derived minerals may e.g. origin from liquid milk, such as skim milk, partially skimmed milk and/or whole milk, or from milk powder, such as e.g. skim milk powder and/or whole milk powder.

In a preferred embodiment, the food ingredient comprises 5-20% by dry weight of vegetable oil, more preferably 8-15% by dry weight of vegetable oil. The vegetable oil may be any suitable vegetable oil, preferably palm oil.

### Process for producing a food ingredient

In another aspect the invention relates to a method of producing the food ingredient of the invention. Accordingly, the invention relates to a method for producing the food ingredient according to the invention, the method comprising:
a) providing an aqueous composition comprising 30-80% by weight of non-fat milk solids including lactose,
b) adding a beta-galactosidase to the composition, wherein the beta-galactosidase is one or more enzymes with enzymatic activity of enzyme class EC 3.2.1.23, which catalyses the hydrolysis of terminal non-reducing beta-D-galactose residues in beta-D-galactosides, as well as transgalactosylation by transferring a galactose moiety of a beta-D-galactoside to another sugar molecule,
c) allowing the beta-galactosidase to react for 10-240 minutes at 50-65°C; and
d) heat treating the composition after step c) at a temperature of 70-150°C for 10-150 seconds.

An aqueous composition comprising 30-80% by weight of non-fat milk solids including lactose may be provided in any suitable way known in the art, e.g. by concentrating milk, such as e.g. skim milk and/or whole milk, to the desired solids concentration, e.g. by evaporation or filtration, or by dissolving milk powder, such as e.g. skim milk powder and/or whole milk powder, in water to arrive at the desired solids concertation. The aqueous composition provided in step a) preferably comprises between 40 and 65% by weight of non-fat milk solids. The aqueous composition provided in step a) preferably comprises 15-45% lactose by weight, more preferably 20-45% lactose by weight. It has been found that this level of solids and/or lactose facilitates the hydrolysis of lactose and formation of GOS. In a preferred embodiment, the composition further comprises 5-20% by weight of vegetable fat, e.g. palm oil.

A beta-galactosidase is added to the aqueous composition provided in step a). The beta-galactosidase may be any suitable beta-galactosidase. The amount of beta-galactosidase added may depend on the specific enzyme, its level of activity and its formulation. The skilled person will now how to choose the amount according to the desired result. Typically, the amount will be in the order of 0.5-2% by weight of lactose in the aqueous composition. The aqueous composition may be at the desired reaction temperature when the beta-galactosidase is added, or it may be heated to the reaction temperature following the addition of the beta-galactosidase. In step b), the beta-galactosidase is allowed to react with the lactose in the aqueous composition to hydrolyse the lactose and produce GOS. The enzyme is allowed to react for 10-240 minutes at 50-65°C. It has been found that this temperature range facilitates the hydrolysis of lactose and formation of GOS.

After the beta-galactosidase has been allowed to react in step c), the composition is heat treated at 70-150°C for 10-150 seconds, preferably at 72-130°C for 10-120 seconds. The purpose of the heat treatment is to prevent substantial degradation of the GOS by further reaction of the beta-galactosidase, as well as ensuring the microbiological stability of the product, without creating excessive denaturation of protein or formation of Maillard reaction products. The food ingredient may be used directly as it is after the heat treatment, or it may e.g. be packed, stored and/or distributed for subsequent use. If the food ingredient is not spray dried, the heat treatment of step d) is preferably conducted at 140-145°C for 10-30 seconds.

In a preferred embodiment, the method comprises spray drying the composition after the heat treatment of step d). It has been found that the heat treatment may not in itself necessarily completely inactivate the beta-galactosidase, but in combination with subsequent spray drying, it ensures full inactivation of the beta-galactosidase. In this way, a more severe heat treatment that would completely inactivate the beta-galactosidase, but also create unnecessary protein denaturation and formation Maillard products, can be avoided by spray drying the composition after the heat treatment to produce a dry powdered food ingredient. When the heat treatment is followed by spray drying, it is preferably conducted at 80-90°C for 10-30 second. The powdered food ingredient may further be packed and distributed, or used directly in the production of a food product, e.g. a cocoa and/or malt beverage product.

In a specific embodiment, the invention relates to a process for producing the food ingredient according to the invention, the process comprising:
a) concentrating skim milk to a solids concentration of 30-80% by weight;
b) adding a beta-galactosidase to the concentrated skim milk;
c) allowing the beta-galactosidase to react for 10-240 minutes at 50-65°C;
d) heat treating the composition after step c) at 80-90°C for 10-30 seconds; and
e) spray drying the composition.

In another specific embodiment, the invention relates to a process for producing the food ingredient according to the invention, the process comprising:
a) providing an aqueous composition comprising 30-80% by weight of non-fat milk solids including lactose,
b) adding a beta-galactosidase to the composition
c) allowing the beta-galactosidase to react for 10-240 minutes at 50-65°C; and
d) heat treating the composition after step c) at 140-145°C for 10-30 seconds.

### Cocoa and/or malt beverage powder

Also disclosed herein for illustration purposes but not forming part of the present invention is a cocoa and/or malt beverage powder comprising: 10-35% by weight of non-fat milk solids; 0-65% by weight of malt extract solids; 0-15% by weight of cocoa solids; 6-15% by weight of vegetable oil and/or milk fat; 0-25% by weight of sucrose; 1-6% by weight of glucose; 0.1-5% by weight of lactose; and 3-10% by weight of GOS.

Non-fat milk solids may be derived from any suitable milk source, such as e.g. liquid milk, e.g. skim milk and/or whole milk, and/or milk powder, e.g. skim milk powder and/or whole milk powder. If liquid milk is used, it may be concentrated, e.g. by evaporation or filtration. Malt extract solids may be in any suitable form, e.g. in the form of a powdered or liquid extract of malted barley and/or wheat. Cocoa solids may be from any suitable cocoa source, e.g. in the form of cocoa powder, cocoa mass and/or cocoa butter. Vegetable oil may be any suitable vegetable oil, preferably palm oil. Milk fat may be derived from any suitable milk source, such as e.g. liquid milk or cream, e.g. skim milk, cream, and/or whole milk, and/or milk powder, e.g. skim milk powder, cream powder, and/or whole milk powder, and/or may be in the form of butter, butter oil and/or anhydrous milk fat. If liquid milk or cream is used, it may be concentrated, e.g. by evaporation or filtration.

Glucose, lactose and GOS are preferably derived from the food ingredient of the present invention. A cocoa and/or malt beverage powder of the present disclosure preferably comprises 10-30% by dry weight of the food ingredient of the invention, more preferably 15-25% by dry weight of the food ingredient of the invention. Preferably, the cocoa and/or malt beverage powder comprises 2-4% by weight of glucose; 0.1-2% by weight of lactose; and 5-10% by weight of GOS.

A cocoa and/or malt beverage powder of the present disclosure may comprise sucrose. Due to the amounts of lactose, glucose and GOS present in the product, the amount of sucrose may be reduced as compared to a conventional cocoa and/or malt beverage powder while retaining an acceptable taste, sweetness and texture. Preferably, the cocoa and/or malt beverage product of the present disclosure does not comprise sucrose. Also preferably, a cocoa and/or malt beverage powder of the present disclosure comprises 1-25% by weight of sucrose, more preferably 5-20% by weight of sucrose.

A cocoa and/or malt beverage powder of the present disclosure may comprise any other suitable ingredients known in the art, such as e.g. vitamins, minerals, buffer salts, emulsifiers and stabilizers.

A cocoa and/or malt beverage powder of the present disclosure may be produced by any suitable method known in the art. The ingredients may e.g. be in powder form and mixed in the dry state, some or all ingredients may be mixed in aqueous solution/suspension and subsequently dried to a powder, or some or all powdered ingredients may e.g. be co-agglomerated to produce a powder with improved solubility.

Preferably, the present disclosure relates to a cocoa and malt beverage powder comprising 15-35% by weight of non-fat milk solids; 20-50% by weight of malt extract solids; 5-15% by weight of cocoa solids; 6-15% by weight of vegetable oil and/or milk fat; 0-25% by weight of sucrose; 1-6% by weight of glucose; 0.1-5% by weight of lactose; and 3-10% by weight of galactooligosaccharide.

### Liquid cocoa and/or malt beverage product

Also disclosed herein for illustration purposes but not forming part of the present invention is a liquid cocoa and/or malt beverage product comprising: 3-10% by weight of non-fat milk solids; 1-6% by weight of malt extract solids; 0-5% by weight of cocoa solids; 0.3-5% by weight of vegetable oil and/or milk fat; 0-5% by weight of sucrose; 0.4-1.5% by weight of glucose; 0.01-1% by weight of lactose; and 0.5-5% by weight of GOS.

A liquid cocoa and/or malt beverage product is a cocoa and/or malt beverage product in liquid form which is suitable to be consumed without addition of liquid. It may e.g. be packed in a container from which it can be consumed directly upon opening.

Non-fat milk solids may be derived from any suitable milk source, such as e.g. liquid milk, e.g. skim milk and/or whole milk, and/or milk powder, e.g. skim milk powder and/or whole milk powder. If liquid milk is used, it may be concentrated, e.g. by evaporation or filtration. Malt extract solids may be in any suitable form, e.g. in the form of a powdered or liquid extract of malted barley and/or wheat. Cocoa solids may be from any suitable cocoa source, e.g. in the form of cocoa powder, cocoa mass and/or cocoa butter. Vegetable oil may be any suitable vegetable oil, preferably palm oil. Milk fat may be derived from any suitable milk source, such as e.g. liquid milk or cream, e.g. skim milk, cream, and/or whole milk, and/or milk powder, e.g. skim milk powder, cream powder, and/or whole milk powder, and/or may be in the form of butter, butter oil and/or anhydrous milk fat. If liquid milk or cream is used, it may be concentrated, e.g. by evaporation or filtration.

Glucose, lactose and GOS are preferably derived from the food ingredient of the present invention. A liquid cocoa and/or malt beverage product of the present disclosure preferably comprises 10-30% by dry weight of the food ingredient of the invention, more preferably 15-25% by dry weight. Preferably, the liquid cocoa and/or malt beverage product comprises 0.5-1% by weight of glucose; 0.01-0.5% by weight of lactose; and 1-4% by weight of GOS.

A liquid cocoa and/or malt beverage product of the present disclosure may comprise sucrose. Due to the amounts of lactose, glucose and GOS present in the product, the amount of sucrose may be reduced as compared to a conventional cocoa and/or malt beverage powder while retaining an acceptable taste, sweetness and texture. Preferably, a cocoa and/or malt beverage product of the present disclosure does not comprise sucrose. Preferably, a cocoa and/or malt beverage powder of the present disclosure comprises 0.1-5% by weight of sucrose, more preferably 1-3% by weight of sucrose.

A liquid cocoa and/or malt beverage product of the present disclosure may comprise any other suitable ingredients known in the art, such as e.g. vitamins, minerals, buffer salts, emulsifiers and stabilizers.

A liquid cocoa and/or malt beverage product of the present disclosure may be produced in any suitable way known in the art. The ingredients may be in liquid and/or powdered form and may be mixed into an aqueous solution/suspension, e.g. with the addition of water to achieve the desired concentration of ingredients. A food ingredient of the invention is preferably added in liquid form, but may also be added in powdered form.

A food ingredient of the invention may form the basis of producing a liquid cocoa and/or malt beverage product of the present disclosure. For example, a food ingredient may be produced by the method of the invention, and after the beta-galactosidase has been allowed to react in step c), the remaining ingredients of the liquid cocoa and/or malt beverage product may be added to the ingredient and the total composition may be heat treated according to step d) of the method of the invention, preferably at 140-145°C for 10-30 seconds.

Preferably, the present disclosure relates to a liquid cocoa and/or malt beverage product comprising: 3-10% by weight of non-fat milk solids; 1-6% by weight of malt extract solids; 1-5% by weight of cocoa solids; 0.3-5% by weight of vegetable oil and/or milk fat; 0-5% by weight of sucrose; 0.4-1.5% by weight of glucose; 0.01-1% by weight of lactose; and 0.5-5% by weight of GOS.

### EXAMPLES

### Example 1

Skimmed milk powder (MSK) was mixed with water to form a slurry with 60% total solids content at 60°C. 1% of a beta-galactosidase with transgalactosylase activity was added to the MSK slurry. The reaction was allowed to proceed for duration of 60 min. Pasteurization at 85°C for 18 seconds was applied to pasteurize the milk and partially inactivate enzymes before spray-drying to form dried skimmed milk powder. It was demonstrated that with spray drying, it was possible to completely inactivate the enzymes and no residual enzyme activity was detected in the final spray dried powder. The composition of the skim milk powder and the enzyme treated powder is shown in table 1.

Table 1. Composition of skim milk powder before and after treatment.

| **% by weight** | **MSK before treatment** | **Enzyme reacted MSK** |
|---|---|---|
| **Glucose** | 0 | 11.6 |
| **Lactose** | 52.4 | 3.0 |
| **Galactose** | 0 | 1.8 |
| **GOS** | 0 | 37.0 |
| **Total Sugars** | 52.4 | 16.4 |
| **Sugar Reduction** | NA | 68.7% |

### Example 2: Preparation of cocoa and malt beverage powder with enzyme-treated

### skimmed milk powder (not according to the present invention)

The enzyme treated skimmed milk powder produced in example 1 was used in the preparation of a cocoa malt beverage powder comprising 20% skimmed milk powder, the composition is given in table 2. A reference cocoa and malt beverage powder was produced in the same way with the same composition, except that untreated skim milk powder was used instead of enzyme treated skim milk powder. The sugar composition of the product and the reference is given in table 3.

**Table 2. Composition of cocoa and malt beverage powder with enzyme-treated skimmed milk powder of example 2.**

| | % by weight of dry matter |
|---|---|
| Malt Extract | 40 |
| Palm Oil | 12 |
| Sucrose | 15 |
| Skimmed Milk Powder | 20 |
| Cocoa Powder | 9.5 |
| Dicalcium Phosphate | 1 |

**Table 3. Sugar composition of cocoa and malt beverage powder with enzyme-treated skimmed milk powder and reference of example 2.**

| % by weight of dry matter | Reference cocoa and malt beverage powder | Cocoa and malt beverage powder with enzyme-treated skimmed milk powder |
|---|---|---|
| Sucrose | 15.6 | 15.6 |
| Fructose | 0.3 | 0.3 |
| Glucose | 2.9 | 5.4 |
| Galactose | 0 | 0.6 |
| Lactose | 10.7 | 0.6 |
| Maltose | 10.3 | 10.3 |
| GOS | 0.0 | 6.8 |
| Total sugars | 39.8 | 32.8 |

The cocoa and malt beverage powder with enzyme-treated skimmed milk powder and the reference cocoa and malt beverage powder were compared by a sensory panel. No significant differences in sweetness and body were found.

### Example 3: Liquid cocoa and malt beverage product (not according to the present invention)

The enzyme treated skimmed milk powder produced in example 1 was used in the preparation of a liquid cocoa malt beverage product with the composition given in table 4. A reference liquid cocoa and malt beverage product was produced in the same way with the same composition, except that untreated skim milk powder was used instead of enzyme treated skim milk powder. The sugar composition of the product and the reference is given in table 5.

**Table 4. Composition of cocoa and malt beverage powder with enzyme-treated skimmed milk powder of example 2.**

| | % by weight |
|---|---|
| Malt Extract | 4 |
| Palm Oil | 3 |
| Sucrose | 4 |
| Skimmed Milk Powder | 4 |
| Cocoa Powder | 3 |
| Stabilizer | 0.5 |

**Table 3. Sugar composition of cocoa and malt beverage powder with enzyme-treated skimmed milk powder and reference of example 2.**

| % by weight | Reference liquid cocoa and malt beverage product | Liquid cocoa and malt beverage powder with enzyme-treated skimmed milk powder |
|---|---|---|
| Sucrose | 4.1 | 4.1 |
| Fructose | 0.0 | 0.0 |
| Glucose | 0.3 | 0.8 |
| Galactose | 0.0 | 0.1 |
| Lactose | 2.1 | 0.1 |
| Maltose | 1.0 | 1.0 |
| GOS | 0.0 | 1.4 |
| Total sugars | 7.5 | 6.1 |

The cocoa and malt beverage powder with enzyme-treated skimmed milk powder and the reference cocoa and malt beverage powder were compared by a sensory panel. No significant differences in sweetness and body were found.

## Claims

1. A food ingredient comprising:
20-40 % by dry weight of milk protein; and
35-55% by dry weight of a mixture of lactose, glucose, galactose and galactooligosaccharide, said mixture containing: 1-14% % by weight of lactose, 15-29% by weight of glucose, 1-8% by weight of galactose and 70-90% by weight of galactooligosaccharide.

2. A food ingredient according to claim 1 further comprising 0.5-30% by dry weight of milk fat and/or mineral derived from milk.

3. A food ingredient according to any one of claims 1 or 2 further comprising 5-20% by dry weight of vegetable oil.

4. A method for producing a food ingredient according to claims 1-3, the method comprising:
a) providing an aqueous composition comprising 30-80% by weight of non-fat milk solids including lactose,
b) adding a beta-galactosidase to the composition, wherein the beta-galactosidase is one or more enzymes with enzymatic activity of enzyme class EC 3.2.1.23, which catalyses the hydrolysis of terminal non-reducing beta-D-galactose residues in beta-D-galactosides, as well as transgalactosylation by transferring a galactose moiety of a beta-D-galactoside to another sugar molecule,
c) allowing the beta-galactosidase to react for 10-240 minutes at 50-65°C; and
d) heat treating the composition after step c) at a temperature of 70-150°C for 10-150 seconds.

5. The method of claim 4 wherein the aqueous composition provided in step a) comprises 15-45% of lactose by weight.

6. The method of any one of claims 4 or 5 further comprising spray drying the composition after step d).

7. The method of any one of claims 4-6 wherein skim milk powder is dissolved in water to provide the composition of step a).

8. The method of any one of claims 4-6 wherein skim milk is concentrated by evaporation and/or filtration to provide the composition of step a).

## Patentansprüche

1. Nahrungsmittelbestandteil, umfassend:
zu 20-40 % Trockengewicht Milcheiweiß; und
zu 35-55 % Trockengewicht eine Mischung aus Laktose, Glukose, Galaktose und Galaktooligosaccharid, wobei die Mischung enthält: zu 1-14 Gew.-% Laktose, zu 15-29 Gew.-% Glukose, zu 1-8 Gew.-% Galaktose und zu 70-90 Gew.-% Galaktooligosaccharid.

2. Nahrungsmittelbestandteil nach Anspruch 1, ferner umfassend zu 0,5-30 % Trockengewicht Milchfett und/oder aus Milch gewonnene Mineralien.

3. Nahrungsmittelbestandteil nach einem der Ansprüche 1 oder 2, ferner umfassend zu 5-20 % Trockengewicht Pflanzenöl.

4. Verfahren zum Herstellen eines Nahrungsmittelbestandteils nach den Ansprüchen 1 bis 3, das Verfahren umfassend:
a) Bereitstellen einer wässrigen Zusammensetzung, umfassend zu 30-80 Gew.-% fettfreie Milchfeststoffe einschließlich Laktose,
b) Hinzufügen einer Beta-Galaktosidase zu der Zusammensetzung, wobei die Beta-Galaktosidase ein oder mehrere Enzyme mit enzymatischer Aktivität der Enzymklasse EC 3.2.1.23 ist, die die Hydrolyse von terminalen nichtreduzierenden Beta-D-Galaktoseresten in Beta-D-Galaktosiden sowie eine Transgalaktosylierung durch Übertragen eines Galaktoseanteils eines Beta-D-Galaktosids auf ein anderes Zuckermolekül katalysiert,
c) Reagierenlassen der Beta-Galaktosidase 10-240 Minuten lang bei 50-65 °C; und
d) Wärmebehandeln der Zusammensetzung nach Schritt c) 10-150 Sekunden lang bei einer Temperatur von 70-150 °C.

5. Verfahren nach Anspruch 4, wobei die in Schritt a) bereitgestellte wässrige Zusammensetzung zu 15-45 Gew.-% Laktose umfasst.

6. Verfahren nach einem der Ansprüche 4 oder 5, ferner umfassend ein Sprühtrocknen der Zusammensetzung nach Schritt d).

7. Verfahren nach einem der Ansprüche 4 bis 6, wobei Magermilchpulver in Wasser gelöst wird, um die Zusammensetzung aus Schritt a) bereitzustellen.

8. Verfahren nach einem der Ansprüche 4 bis 6, wobei Magermilch durch Verdampfung und/oder Filtration konzentriert wird, um die Zusammensetzung aus Schritt a) bereitzustellen.

## Revendications

1. Ingrédient alimentaire comprenant :
20 à 40 % en poids sec de protéines de lait ; et
35 à 55 % en poids sec d'un mélange de lactose, de glucose, de galactose et de galactooligosaccharide, ledit mélange contenant : 1 à 14 % en poids de lactose, 15 à 29 % en poids de glucose, 1 à 8 % en poids de galactose et 70 à 90 % en poids de galactooligosaccharide.

2. Ingrédient alimentaire selon la revendication 1, comprenant en outre 0,5 à 30 % en poids sec de matière grasse laitière et/ou de minéraux dérivés du lait.

3. Ingrédient alimentaire selon l'une quelconque des revendications 1 ou 2, comprenant en outre 5 à 20 % en poids sec d'huile végétale.

4. Procédé de production d'un ingrédient alimentaire selon les revendications 1 à 3, le procédé comprenant :
a) la fourniture d'une composition aqueuse comprenant 30 à 80 % en poids de solides laitiers non gras, y compris le lactose,
b) l'ajout d'une bêta-galactosidase à la composition, dans lequel la bêta-galactosidase est une ou plusieurs enzymes ayant une activité enzymatique de la classe d'enzymes EC 3.2.1.23, qui catalyse l'hydrolyse de résidus bêta-D-galactose terminaux non réducteurs dans des bêta-D-galactosides, ainsi que la transgalactosylation par transfert d'une partie galactose d'un bêta-D-galactoside à une autre molécule de sucre,
c) le fait de laisser la bêta-galactosidase réagir pendant 10 à 240 minutes à 50 à 65 °C ; et
d) le traitement thermique de la composition après l'étape c) à une température de 70 à 150 °C pendant 10 à 150 secondes.

5. Procédé selon la revendication 4, dans lequel la composition aqueuse fournie à l'étape a) comprend 15 à 45 % de lactose en poids.

6. Procédé selon l'une quelconque des revendications 4 à 5, comprenant en outre le séchage par pulvérisation de la composition après l'étape d).

7. Procédé selon l'une quelconque des revendications 4 à 6, dans lequel le lait écrémé en poudre est dissous dans l'eau pour obtenir la composition de l'étape a).

8. Procédé selon l'une quelconque des revendications 4 à 6, dans lequel le lait écrémé est concentré par évaporation et/ou filtration pour obtenir la composition de l'étape a).
